# EUROPÄISCHE PATENTSCHRIFT

(11) **EP 2 446 813 B1**
(45) Veröffentlichungstag und Bekanntmachung des Hinweises auf die Patenterteilung: **07.03.2018**
(21) Anmeldenummer: 11184914.7
(22) Anmeldetag: 12.10.2011
(51) Int. Cl.: A61B 3/16, A61B 3/00

(54) **Ophthalmologisches Analysegerät**
Ophthalmologic Device for Analysis
Appareil ophthalmique pour l'analyse

(30) Priorität: 08.09.2011 DE 102011082363; 28.10.2010 DE 102010049632
(43) Veröffentlichungstag der Anmeldung: 02.05.2012
(73) Patentinhaber: Oculus Optikgeräte GmbH, 35582 Wetzlar-Dutenhofen (DE)
(72) Erfinder: Köst, Gert, 30159 Hannover (DE)
(74) Vertreter: advotec.

(56) Entgegenhaltungen:
- EP-A1- 1 779 771
- EP-A1- 2 095 761
- EP-A1- 2 113 192
- DE-C2- 3 990 085
- US-A- 4 834 527
- US-A- 5 002 056
- US-A- 5 751 395
- US-A- 6 053 867
- US-A- 6 120 444
- US-A1- 2005 174 534

## Beschreibung

Die Erfindung betrifft ein ophthalmologisches Analysegerät zur Messung eines intraokularen Drucks in einem Auge, mit einem Beleuchtungssystem, wobei das Analysegerät eine Betätigungsvorrichtung aufweist, mit der ein Luftstoß zur Verformung einer Hornhaut auf das Auge, in Richtung einer optischen Achse des Auges, aufgebracht werden kann, wobei das Beleuchtungssystem zumindest eine Beleuchtungsvorrichtung umfasst, mit der die Hornhaut des Auges mit einem Lichtspalt beleuchtet werden kann, derart, dass in einer mit der optischen Achse übereinstimmenden Beleuchtungsebene ein Schnittbild erzeugt werden kann.

Derartige Analysesysteme können beispielsweise als sogenannte Nonkontakt-Tonometer ausgebildet sein, die eine Hornhaut eines Auges berührungsfrei durch Aufbringen eines Luftstoßes mittels einer Betätigungsvorrichtung verformen können. Die Betätigungsvorrichtung kann eine Düse umfassen, die in etwa in Richtung einer Sehachse des Auges positioniert wird. Zur Messung eines intraokularen Drucks in dem Auge ist es notwendig, eine durch den Luftstoß bewirkte Verformung zu beobachten. So ist es bekannt, diese Verformung mit einem Beobachtungssystem aufzunehmen. Das Beobachtungssystem kann beispielsweise einen Zeitpunkt eines Einklappens und eines Ausklappens, sowie eine ebene Applanation der Hornhaut durch den Luftstoß erfassen. Das Beobachtungssystem ist regelmäßig aus einer Kameravorrichtung gebildet, wobei während der Verformung der Hornhaut das Auge mit einem Beleuchtungssystem so beleuchtet wird, dass aus erfassten Lichtreflektionen der Hornhaut die vorgenannten Verformungsstadien abgeleitet werden können. Auch sind Beleuchtungssysteme bekannt, bei denen das Auge so beleuchtet wird, dass Schnittbilder der unverformten und der verformten Hornhaut mittels der Kameravorrichtung aufgenommen werden können. In diesem Fall ist eine Beleuchtungsvorrichtung vorgesehen, die in Richtung der optischen Achse des Auges einen Lichtspalt auf die Hornhaut projiziert. Ein von dem Lichtspalt beleuchteter Bereich der Hornhaut wird als ein Schnittbild sichtbar, welches von einer schräg neben dem Auge angeordneten Kameravorrichtung aufgenommen werden kann. Dabei besteht die Problematik, dass eine Beleuchtung der Hornhaut bzw. eine die Sehachse des Auges schneidende Beleuchtungsebene im Auge durch eine Position der Düse der Betätigungsvorrichtung auf der Sehachse behindert wird. Dem wird zwar dadurch entgegengetreten, dass ein Bereich um eine Düsenöffnung herum transparent ausgebildet ist, und dass der Lichtspalt durch die Düsenöffnung auf das Auge projiziert wird, jedoch stellt dies keine befriedigende Lösung dar, da der Lichtspalt nicht direkt und somit nicht störungsfrei auf das Auges projiziert werden kann. Weiter ist es notwendig die Betätigungsvorrichtung mit Ausnahme der Düse außerhalb der optischen Achse des Auges anzuordnen, was einen konstruktiv erhöhten Aufwand erfordert.

Aus der DE 39 90 085 C2 ist ein Nonkontakt-Tonometer mit einem Beleuchtungssystem bekannt, welches zur Projektion einer Zielmarke auf die Hornhaut eines Auges ausgebildet ist. Das Beleuchtungssystem umfasst zwei Beleuchtungsvorrichtungen mit jeweils einer LED und einer Blende, welche in einem Winkel α relativ zu einer optischen Achse eines Auges angeordnet sind. Die jeweiligen Zielmarken der Beleuchtungsvorrichtungen sind punktförmig beziehungsweise kreisförmig ausgebildet.

Die EP 1 779 771 A1 offenbart ein Nonkontakt-Tonometer mit einem Beleuchtungssystem mit einer Lichtquelle, welche in einem Winkel α relativ zu einer optischen Achse eines Auges angeordnet ist. Auch ist ein weiteres Beleuchtungssystem vorgesehen, welches zur Messung einer Hornhautdicke verwendet wird.

Die US 2005/174534 A1 beschreibt ein Nonkontakt-Tonometer mit einem Beleuchtungssystem, welches vier Beleuchtungsvorrichtungen aufweist. Zwei der Beleuchtungsvorrichtungen sind in einer gemeinsamen horizontalen Ebene in einem Winkel α relativ zu einer optischen Achse eines Auges angeordnet. Die Beleuchtungsvorrichtungen dienen zur Bestimmung einer Topographie einer Hornhaut.

Ein weiteres Nonkontakt-Tonometer ist aus der US 6,053,867 bekannt, wobei ein Lichtspalt mittels eines Beleuchtungssystems auf eine Hornhaut projiziert wird. Der Lichtspalt durchdringt die Oberfläche der Hornhaut und wird von einer rückwärtigen Fläche der Hornhaut reflektiert, sodass eine Dicke der Hornhaut bestimmt werden kann. Die Dickenmessung erfolgt mit einer Zeilenkamera, auf der das Reflexionsbild des Lichtspaltes projiziert wird.

Ein aus der US 6,120,444 bekanntes Beleuchtungssystem wird zur direkten, das heisst in der Richtung einer optischen Achse eines Auges ausgerichteten Beleuchtung der Hornhaut in Zusammenspiel mit einer Scheimflugkamera verwendet. Ein Lichtspalt wird entlang der optischen Achse auf die Hornhaut des Auges projiziert, sodass ein Schnittbild in einer mit der optischen Achse übereinstimmenden Beleuchtungsebene erzeugt werden kann.

Bei dem Nonkontakt-Tonometer der US 5,002,056 ist ein Beleuchtungssystem quer zu einer optischen Achse angeordnet und dient zur Beobachtung einer Hornhautverformung. Im Wesentlichen wird hier ein Reflexionsbild einer hier nicht näher beschriebenen Zielmarke beobachtet.

Die US 4,834,527 beschreibt ein Nonkontakt-Tonometer in Art dem der DE 39 90 085 C2 mit Beleuchtungsvorrichtungen, wobei eine im Wesentlichen punktförmige Beleuchtung der Hornhaut eines Auges zur Erzeugung einer detektierbaren Reflexionsmarke erfolgt.

Aus der US 5,751,395 ist eine Beleuchtungsvorrichtung bekannt, deren Strahlengang abschnittsweise in Richtung einer optischen Achse eines Auges ausgerichtet ist.

Ein Analysegerät mit den Merkmalen des Oberbegriffs des Anspruchs 1 ist aus der EP 2 095 761 A1 bekannt. Die EP 2 095 761 A1 beschreibt ein Nonkontakt-Tonometer mit einem Beleuchtungssystem, welches eine Spaltblende aufweist. Über einen Strahlenteilerwürfel wird ein Lichtspalt des Beleuchtungssystems in Richtung und koaxial mit einer optischen Achse des Nonkontakt-Tonometers auf das zu untersuchende Auge projiziert. Insbesondere ist dabei vorgesehen, dass der Lichtspalt genau durch eine Düsenöffnung des Nonkontakt-Tonometers verläuft. Nach einer Ausführungsform des Analysegerätes kann auch eine Scheimpflugkamera zur Aufnahme eines Schnittbildes des Auges vorgesehen sein.

Der vorliegenden Erfindung liegt daher die Aufgabe zugrunde, ein ophthalmologisches Analysegerät vorzuschlagen, mit dem eine Beleuchtungsqualität bei für einen Patienten angenehmeren Untersuchungsbedingungen verbessert werden kann.

Diese Aufgabe wird durch ein Analysegerät mit den Merkmalen des Anspruchs 1 gelöst.

Bei dem erfindungsgemäßen ophthalmologischen Analysegerät zur Messung eines intraokularen Drucks in einem Auge, mit einem Beleuchtungssystem, weist das ophthalmologische Analysegerät eine Betätigungsvorrichtung auf, mit der ein Luftstoß zur Verformung einer Hornhaut auf das Auge, in Richtung einer optischen Achse des Auges, aufgebracht werden kann, wobei das Beleuchtungssystem zumindest eine Beleuchtungsvorrichtung umfasst, mit der die Hornhaut des Auges mit einem Lichtspalt beleuchtet werden kann, derart, dass in einer mit der optischen Achse übereinstimmenden Beleuchtungsebene ein Schnittbild erzeugt werden kann, wobei die Beleuchtungsvorrichtung so ausgebildet und seitlich relativ zur Betätigungsvorrichtung angeordnet ist, dass ein auf das Auge ausgerichteter Beleuchtungsstrahlengang der Beleuchtungsvorrichtung in einem Winkel α relativ zur optischen Achse bzw. Sehachse des Auges angeordnet ist.

Demnach ist vorgesehen, die Beleuchtungsvorrichtung so seitlich neben einer Düse der Betätigungsvorrichtung anzuordnen, dass dennoch in einem Bereich der in der Sehachse liegenden Beleuchtungsebene ein Schnittbild im Auge erzeugt werden kann, ein Strahlengang der Beleuchtungsvorrichtung jedoch nicht mehr von der Betätigungsvorrichtung bzw. der Düse behindert wird. Der Lichtspalt bzw. der Beleuchtungsstrahlengang muss dabei in der mit der optischen Achse übereinstimmenden Beleuchtungsebene liegen, damit ein Maximum einer Verformung der Hornhaut überhaupt erfasst werden kann. Dies wird dadurch möglich, dass die Beleuchtungsebene in Richtung des Luftstoßes ausgerichtet ist. Eine Qualität der Beleuchtung und damit der Schnittbilder kann durch die seitliche Anordnung der Beleuchtungsvorrichtung spürbar verbessert werden. Weiter wird es möglich die Betätigungsvorrichtung auch vollständig in Richtung der optischen Achse anzuordnen, ohne dass eine beispielsweise gebogene Düsenzuleitung notwendig wäre. Dadurch wird die Betätigungsvorrichtung insgesamt auch kompakter ausbildbar. Ein weiterer Vorteil der seitlich schrägen Anordnung der Beleuchtungsvorrichtung ist, dass eine Blendung einer zu untersuchenden Person gemindert wird, da dass in das Auge einfallende Licht nicht unmittelbar in einen Zentralbereich einer Netzhaut des Auges projiziert wird.

Besonders vorteilhaft ist es, wenn das Beleuchtungssystem zwei Beleuchtungsvorrichtungen umfasst, die die Beleuchtungsebene beleuchten können. So kann sichergestellt werden, dass keine unerwünschte Abschattung von Randbereichen der Hornhaut durch alleine eine Beleuchtungsvorrichtung erfolgt. Auch können noch weiter verbesserte Schnittbilder erzielt werden, da aufgrund gegebenenfalls unterschiedlicher Lichteinfallswinkel der unterschiedlichen Beleuchtungsstrahlengänge der Beleuchtungsvorrichtung eine verbesserte Lichtstreuung innerhalb des optischen Mediums der Hornhaut erzielt werden kann. Auch können auf oder in der Hornhaut befindliche Partikel durch die Verwendung von zwei Beleuchtungsvorrichtungen so beleuchtet werden, dass ein unerwünschter Partikelschatten zumindest teilweise ausgeleuchtet bzw. eliminiert wird. Auch wird durch die Verwendung von zwei derartigen Beleuchtungsvorrichtungen die Möglichkeit eröffnet, ein Schnittbild in einem besonders großen Bereich der Hornhaut zu erzeugen.

Weiter können die Beleuchtungsvorrichtungen koaxial zur optischen Achse angeordnet sein. Aus einer koaxialen, und dadurch relativ auf die optische Achse bezogenen, gegenüberliegenden Anordnung der Beleuchtungsvorrichtungen ergibt sich der Vorteil, dass die Beleuchtungsstrahlengänge besonders gut aufeinander abgestimmt werden können. So ist insbesondere eine Überlagerung der Strahlengänge im Bereich der Hornhaut erforderlich.

Besonders vorteilhaft ist es, wenn die Beleuchtungsvorrichtung eine Filtereinrichtung aufweist, welche Helligkeitsunterschiede eines Schnittbildes kompensieren kann. Helligkeitsunterschiede eines Schnittbildes können beispielsweise dadurch entstehen, dass bei seitlich schräg in die Hornhaut einfallenden Beleuchtungsstrahlengang ein Abschnitt des Beleuchtungsstrahlengangs einen längeren Weg durch die Hornhaut zurücklegt, als ein anderer Abschnitt. Mit Hilfe der Filtereinrichtung kann dann der Teil des Beleuchtungsstrahlengangs, der ein heller erscheinendes Schnittbild bewirkt, so abgedunkelt werden, dass das Schnittbild insgesamt gleichmäßig hell erscheint.

Die Filtereinrichtung kann beispielsweise zumindest einen Verlaufsfilter ausbilden. Mit einem Verlaufsfilter kann besonders einfach eine Abdunklung eines Schnittbildes in gegebenenfalls zu hell erscheinenden Bereichen desselben erzielt werden.

Der Verlaufsfilter kann dabei so ausgebildet sein, dass er den Beleuchtungsstrahlengang mittig abdunkeln kann. Insbesondere wenn zwei Beleuchtungsvorrichtungen verwendet werden, ist es möglich, dass sich im Bereich der sich überschneidenden Beleuchtungsstrahlengänge innerhalb der Hornhaut des Auges besonders helle Bereiche in dem Schnittbild ausbilden. Ein derartiger Verlaufsfilter ermöglicht das Abdunkeln dieser Überschneidungsbereiche.

Alternativ oder ergänzend kann der Verlaufsfilter den Beleuchtungsstrahlengang quer zum Lichtspalt stetig abdunkeln. So kann ein von Hell nach Dunkel reichender Verlauf des Beleuchtungsstrahlengangs quer zu demselben erzielt werden. Mit einem derartigen Verlaufsfilter kann insbesondere der seitlich schräge Einfall des Beleuchtungsstrahlengangs in das Auge berücksichtigt und eventuelle Helligkeitsabweichungen des Schnittbildes korrigiert werden.

Ein Leuchtmittel der Beleuchtungsvorrichtung kann aus zumindest einer Leuchtdiode gebildet sein. Dabei kann die Leuchtdiode in Verbindung mit einer oder mehreren Linsen und/oder Blenden zur Ausbildung des Beleuchtungsstrahlengangs in der Beleuchtungsvorrichtung angeordnet sein. Weiter ist es auch denkbar, mehrere Leuchtdioden in einer Reihenanordnung zur Ausbildung des Lichtspaltes zu verwenden.

Die Beleuchtungsvorrichtung kann auch eine Umlenkeinrichtung, wie zum Beispiel ein Prisma oder einen Spiegel, aufweisen, mittels der der Beleuchtungsstrahlengang in der Beleuchtungsvorrichtung um einen Winkel β umgelenkt werden kann. Der Winkel β kann beispielsweise 90° betragen, so dass ein Gehäuse der Beleuchtungsvorrichtung quer zur Beleuchtungsebene angeordnet werden kann. Durch die Umlenkeinrichtung wird es möglich, die Beleuchtungsvorrichtung bzw. ein Gehäuse derselben so auszubilden bzw. anzuordnen, dass das Analysegerät kompakt ausgebildet werden kann.

Das Analysegerät kann ein Beobachtungssystem umfassen, mit dem die Verformung der Hornhaut beobachtet und aufgenommen werden kann, wobei mit dem Beobachtungssystem Schnittbilder der unverformten und verformten Hornhaut aufgenommen werden können. So wird es möglich mittels einer Analyseeinrichtung eines Analysegeräts aus den Schnittbildern der Hornhaut den intraokularen Druck abzuleiten. Der intraokulare Druck kann dabei besonders genau bestimmt werden, da durch die Verwendung des erfindungsgemäßen Beleuchtungssystems Schnittbilder hoher optischer Qualität gewonnen werden können. Da die Schnittbilder mittels der Analyseeinrichtung hinsichtlich eines Verformungsverlaufs ausgewertet werden, kann folglich der Verformungsverlauf besonders präzise ermittelt werden.

Wenn das Beleuchtungssystem räumlich unabhängig von der Betätigungsvorrichtung ausgebildet ist, kann eine Beeinflussung der Beleuchtungsvorrichtung bzw. des Beleuchtungssystems durch einen gegebenenfalls mechanischen Antrieb der Betätigungsvorrichtung ausgeschlossen werden. Auch kann dann ein Auswechseln oder auch eine Wartung und Kalibrierung des Beleuchtungssystems unabhängig von dem Analysesystem durchgeführt werden, da das Beleuchtungssystem dann besonders einfach ausgetauscht werden kann.

Auch kann eine Beleuchtungsvorrichtung des Beleuchtungssystems relativ zu einer Geräteachse der Betätigungsvorrichtung bzw. die optische Achse des Auges um einen Winkel α schwenkbar ausgebildet sein. So wird es möglich einen Einfallswinkel des Beleuchtungsstrahlengangs relativ zur optischen Achse in das Auge in geeigneter Weise einzustellen. Der Einfallswinkel kann dann auf bestimme Anforderungen einer Messung angepasst werden.

Vorteilhaft ist es, wenn das Beobachtungssystem eine Kameravorrichtung umfasst, mit der die Schnittbilder aufgenommen werden können, und wobei die Kameravorrichtung und das Beleuchtungssystem so angeordnet sind, dass die Kameravorrichtung und das Schnittbild in einer Scheimpfluganordnung nach der Scheimpflugschen-Regel angeordnet sind. So können dann mittels der Kameravorrichtung Aufnahmen der Schnittbilder gewonnen werden, die entzerrt sind. Dies ermöglicht eine unmittelbare Messung von Längen und Positionen aus den Schnittbildern, ohne dass noch weitere, korrigierende Berechnungen notwendig wären.

Um ein Auge bei einer möglichen Kollision mit der Betätigungsvorrichtung vor Verletzungen durch eine Düse der Betätigungsvorrichtung zu schützen, kann die Betätigungsvorrichtung eine transparente Platte aufweisen, in der eine Öffnung zum Ausbringen des Luftstoßes ausgebildet ist, wobei ein Beleuchtungsstrahlengang des Beleuchtungssystem die Platte durchdringen kann. So kann die transparente Platte die Düse so weit umgeben und eine Düsenöffnung ausbilden, dass eine Verletzung des Auges durch spitze Bauteile verhindert wird. Je nach Größe der Platte kann der Beleuchtungsstrahlengang bzw. die Beleuchtungsstrahlengänge des Beleuchtungssystems durch die transparente Platte hindurch auf das Auge gerichtet sein.

Weiter kann das Analysegerät so ausgebildet sein, dass das Beobachtungssystem zusammen mit dem Beleuchtungssystem um eine Geräteachse der Betätigungsvorrichtung bzw. die optische Achse des Auges gedreht werden kann. Dadurch wird es möglich, Schnittbilder der jeweils verschiedenen Drehwinkel zu gewinnen, woraus mittels der Analyseeinrichtung ein dreidimensionales Modell des betreffenden Bereiches des Auges aus den Schnittbildern abgleitet werden kann. Insbesondere die durch das Beleuchtungssystem erst ermöglichte, kompakte Ausbildung der Betätigungsvorrichtung erleichtert die Ausbildung eines derartigen Analysegerätes. So können das Beobachtungssystem und das Beleuchtungssystem ungehindert um die Geräteachse rotieren, da die Betätigungsvorrichtung entlang der optischen Achse ausgebildet werden kann. Nachfolgend wird eine bevorzugte Ausführungsform der Erfindung unter Bezugnahme auf die beigefügten Zeichnungen näher erläutert.

Es zeigen:
- **Fig. 1**: eine schematisch Darstellung eines Analysegerätes in einer Draufsicht;
- **Fig. 2**: eine schematische Darstellung des Analysegerätes in einer Seitenansicht;
- **Fig. 3**: eine perspektivische Ansicht eines Beleuchtungssystems;
- **Fig. 4**: eine perspektivische Ansicht des Beleuchtungssystems zusammen mit einer Betätigungsvorrichtung.

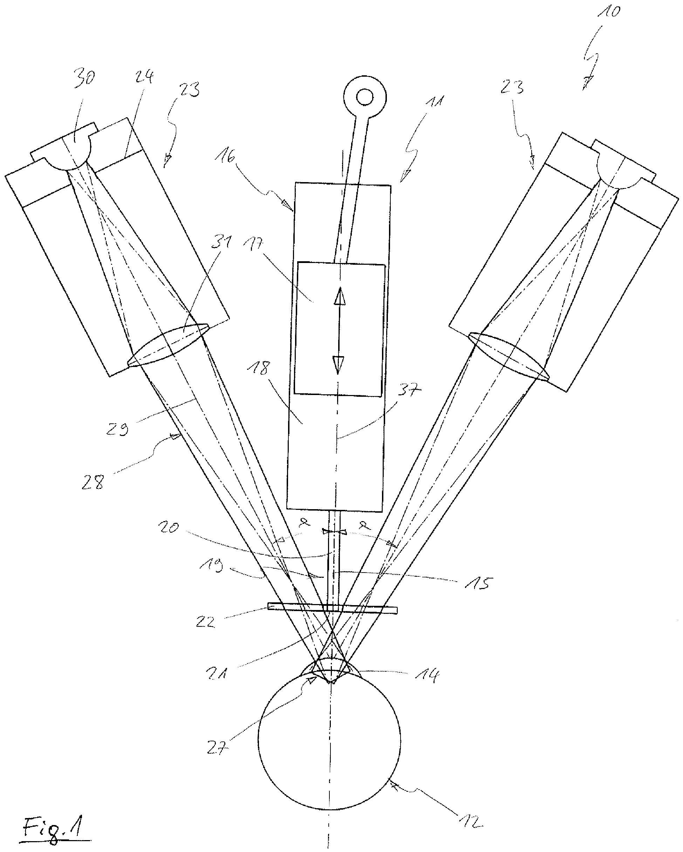

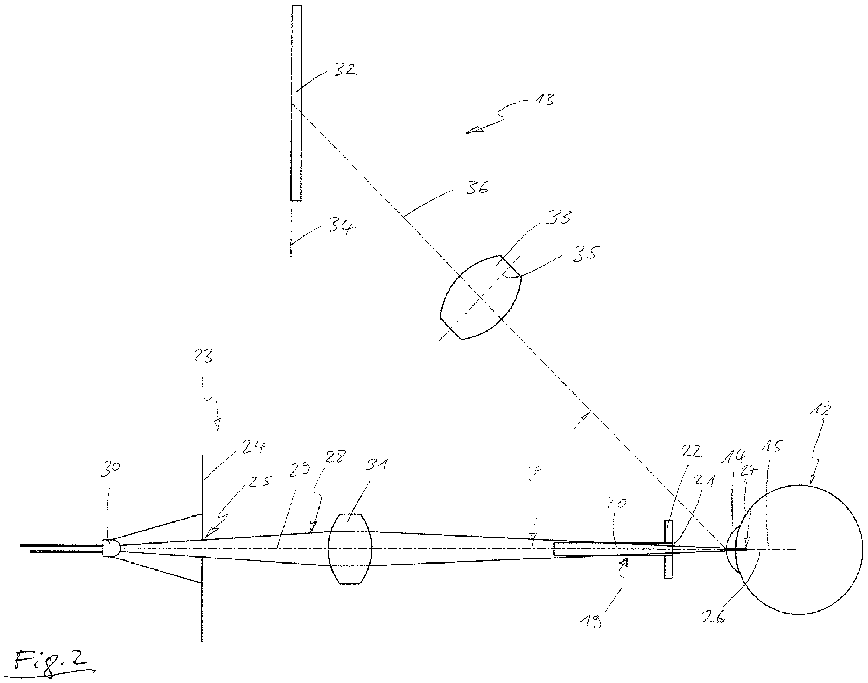

Eine Zusammenschau der **Fig. 1** und **2** zeigt ein Beleuchtungssystem 10 mit einer teilweise dargestellten Betätigungsvorrichtung 11 eines hier nicht gezeigten Analysegerätes sowie ein Auge 12. Weiter ist insbesondere in **Fig. 2** ein Beobachtungssystem 13 des Analysegerätes ersichtlich. Das Analysegerät dient zu einer Messung eines intraokularen Drucks in dem Auge 12, wobei mit der Betätigungsvorrichtung 11 ein Luftstoß zur Verformung einer Hornhaut 14 des Auges 12 auf das Auge 12, in Richtung einer optischen Achse 15 des Auges 12 aufgebracht wird. Die Betätigungsvorrichtung 11 ist dabei im Wesentlichen aus einer Kolbenpumpe 16, mit einem hier nicht näher dargestellten Antrieb, und einem Kolben 17, welcher längsbeweglich in einem Zylinder 18 angeordnet ist sowie einer Düse 19 ausgebildet. Die Düse 19 umfasst einen Düsenkanal 20, eine Düsenöffnung 21 und eine transparente Platte 22, in der die Düsenöffnung 21 ausgebildet ist. Die Betätigungseinrichtung 11 ist im Wesentlichen längs der optischen Achse 15 angeordnet.

Das Beleuchtungssystem 10 umfasst zwei Beleuchtungsvorrichtungen 23, die die Hornhaut 14 mit einem mit einer Blende 24 erzeugten Lichtspalt 25 so beleuchten, dass in einer mit der optischen Achse 15 übereinstimmenden Beleuchtungsebene 26 ein Schnittbild 27 erzeugt wird. Ein Beleuchtungsstrahlengang 28 bzw. dessen optische Achse 29 der Beleuchtungsvorrichtung 23 ist in einem Winkel α relativ zur optischen Achse 15 des Auges 12 angeordnet. Die Beleuchtungsvorrichtungen 23 umfassen jeweils im Wesentlichen eine Leuchtdiode 30 als eine Lichtquelle sowie eine Linse 31 zur Anpassung des Beleuchtungsstrahlengangs 28. Die Beleuchtungsvorrichtungen 23 sind so relativ zur Betätigungsvorrichtung 11 angeordnet, dass die jeweiligen Beleuchtungsstrahlengänge 28 durch die transparente Platte 22 hindurch in das Auge 12 fallen und sich im Bereich der Hornhaut 14 gegenseitig überlagern und das Schnittbild 27 erzeugen.

Das Schnittbild 27 wird mittels des Beobachtungssystems 13 aufgenommen, welches hier auch nur schematisch dargestellt ist. Das Beobachtungssystem 13 ist aus einer hier nicht näher dargestellten Kamera mit einem Bildsensor 32 und einem Objektiv 33 gebildet. Der Bildsensor 32 bildet eine Projektionsebene 34 aus, die zusammen mit einer Objektivebene 35 und der Beleuchtungsebene 26 als eine Bildebene nach der Scheimpflugschen-Regel angeordnet sind. Im vorliegenden Beispiel ist eine optische Achse 36 des Beobachtungssystems 13 unter einem Winkel γ relativ zur Beleuchtungsebene 26 angeordnet, wobei die optische Achse 36 mit der optischen Achse 15 in einer Querschnittsebene 37 liegt.

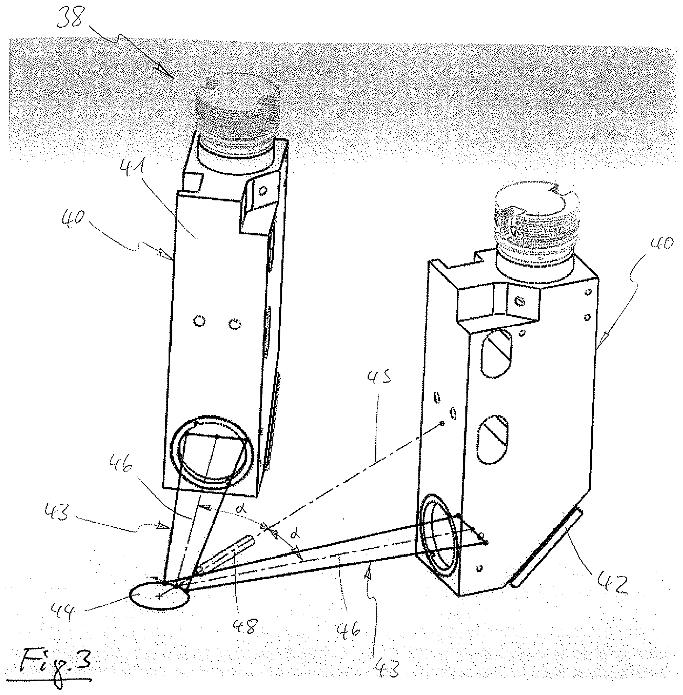

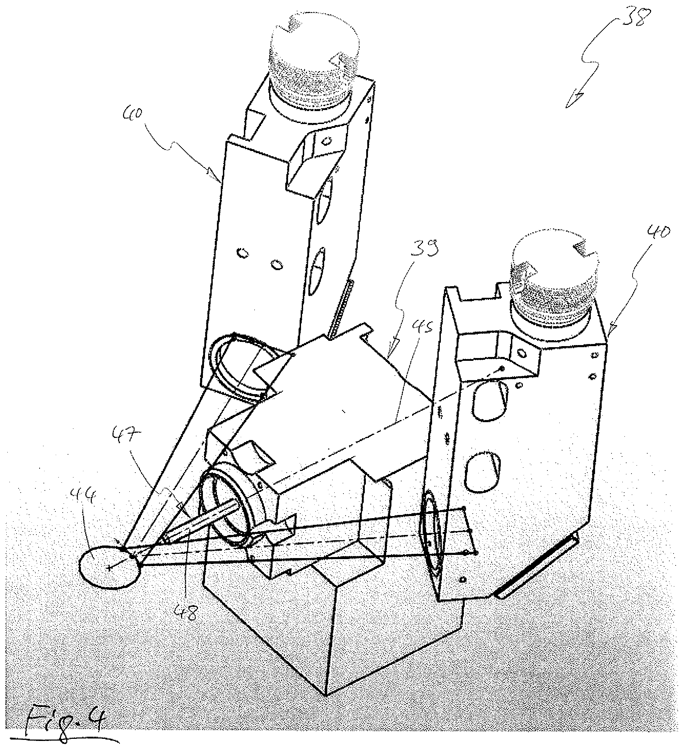

Eine Zusammenschau der **Fig. 3** und 4 zeigt ein Beleuchtungssystem 38 in verschiedenen, perspektivischen Darstellungen, wobei in **Fig. 4** ergänzend eine Betätigungsvorrichtung 39 abgebildet ist. Das Beleuchtungssystem 38 besteht im Wesentlichen aus zwei identischen Beleuchtungsvorrichtungen 40 mit einem Gehäuse 41 und einer hier nicht näher ersichtlichen Lichtquelle sowie einer als Spiegel 42 ausgebildeten Umlenkeinrichtung zur Umlenkung eines Beleuchtungsstrahlengangs 43 auf ein Auge 44. Der Beleuchtungsstrahlengang 43 wird dabei in einem Winkel von ca. 90° umgelenkt. Die Beleuchtungsvorrichtungen 40 sind relativ zu einer optischen Achse 45 des Auges 44 so angeordnet, dass eine optische Achse 46 des Beleuchtungsstrahlengangs 43 einen Winkel α mit der optischen Achse 45 des Auges 44 bildet, so dass die Betätigungsvorrichtung 39 zwischen den beiden Beleuchtungsvorrichtungen 40 noch vor dem Auge 44 mit einer Düse 47 angeordnet werden kann. Ein Düsenkanal 48 der Düse 47 fluchtet dabei mit der optischen Achse 45 des Auges 44.

## Patentansprüche

1. Ophthalmologisches Analysegerät zur Messung eines intraokularen Drucks in einem Auge, mit einem Beleuchtungssystem (10, 38), wobei das Analysegerät eine Betätigungsvorrichtung (11, 39) aufweist, mit der ein Luftstoß zur Verformung einer Hornhaut (14) auf das Auge (22) fluchtend mit der optischen Achse (15, 45) des Auges aufgebracht werden kann, wobei das Beleuchtungssystem zumindest eine Beleuchtungsvorrichtung (23, 40) umfasst, mit der die Hornhaut des Auges mit einem Lichtspalt (25) beleuchtet werden kann, derart, dass in einer mit der optischen Achse übereinstimmenden Beleuchtungsebene (26) ein Schnittbild (27) erzeugt werden kann,
**dadurch gekennzeichnet,**
**dass** die Beleuchtungsvorrichtung so ausgebildet und seitlich relativ zur Betätigungsvorrichtung angeordnet ist, dass ein auf das Auge ausgerichteter Beleuchtungsstrahlengang (28, 43) der Beleuchtungsvorrichtung in einem Winkel α relativ zur optischen Achse (15, 45) angeordnet ist.

2. Analysegerät nach Anspruch 1,
**dadurch gekennzeichnet,**
**dass** das Beleuchtungssystem (10, 38) zwei Beleuchtungsvorrichtungen (23, 40) umfasst, die die Beleuchtungsebene (26) beleuchten können.

3. Analysegerät nach Anspruch 2,
**dadurch gekennzeichnet,**
**dass** die Beleuchtungsvorrichtungen (23, 40) koaxial zur optischen Achse (15, 45) angeordnet sind.

4. Analysegerät nach einem der vorangehenden Ansprüche,
**dadurch gekennzeichnet,**
**dass** die Beleuchtungsvorrichtung (23, 40) eine Filtereinrichtung aufweist, welche Helligkeitsunterschiede eines Schnittbildes (27) kompensieren kann.

5. Analysegerät nach Anspruch 4,
**dadurch gekennzeichnet,**
**dass** die Filtereinrichtung zumindest einen Verlaufsfilter ausbildet.

6. Analysegerät nach Anspruch 5,
**dadurch gekennzeichnet,**
**dass** der Verlaufsfilter den Beleuchtungsstrahlengang (28, 43) mittig abdunkeln kann.

7. Analysegerät nach Anspruch 5 oder 6,
**dadurch gekennzeichnet,**
**dass** der Verlaufsfilter den Beleuchtungsstrahlengang (28, 43) quer zum Lichtspalt (25) stetig abdunkeln kann.

8. Analysegerät nach einem der vorangehenden Ansprüche,
**dadurch gekennzeichnet,**
**dass** ein Leuchtmittel der Beleuchtungsvorrichtung (23, 40) aus zumindest einer Leuchtdiode (30) gebildet ist.

9. Analysegerät nach einem der vorangehenden Ansprüche,
**dadurch gekennzeichnet,**
**dass** die Beleuchtungsvorrichtung (40) eine Umlenkeinrichtung (42) aufweist, mittels der der Beleuchtungsstrahlengang (43) in der Beleuchtungsvorrichtung um einen Winkel β umgelenkt werden kann.

10. Analysegerät nach einem der vorangehenden Ansprüche,
**dadurch gekennzeichnet,**
**dass** das Analysegerät ein Beobachtungssystem (13) umfasst, mit dem die Verformung der Hornhaut beobachtet und aufgenommen werden kann, wobei mit dem Beobachtungssystem Schnittbilder (27) der unverformten und verformten Hornhaut aufgenommen werden können.

11. Analysegerät nach Anspruch 10,
**dadurch gekennzeichnet,**
**dass** das Beleuchtungssystem (10, 38) räumlich unabhängig von der Betätigungsvorrichtung (11, 39) ausgebildet ist.

12. Analysegerät nach Anspruch 10 oder 11,
**dadurch gekennzeichnet,**
**dass** eine Beleuchtungsvorrichtung (23, 40) des Beleuchtungssystems (10, 38) relativ zu einer Geräteachse der Betätigungsvorrichtung (11, 39) bzw. die optische Achse (15, 45) um einem Winkel α schwenkbar ausgebildet ist.

13. Analysegerät nach einem der Ansprüche 10 bis 12,
**dadurch gekennzeichnet,**
**dass** das Beobachtungssystem (13) eine Kameravorrichtung (32, 33) umfasst, mit der die Schnittbilder (27) aufgenommen werden können, und wobei die Kameravorrichtung und das Beleuchtungssystem (10, 38) so angeordnet sind, dass die Kameravorrichtung und das Schnittbild in einer Scheimpfluganordnung angeordnet sind.

14. Analysegerät nach einem der Ansprüche 10 bis 13,
**dadurch gekennzeichnet,**
**dass** die Betätigungsvorrichtung (11, 39) eine transparente Platte (22) aufweist, in der eine Öffnung (21) zum Ausbringen des Luftstoßes ausgebildet ist, wobei ein Beleuchtungsstrahlengang (28, 43) des Beleuchtungssystems (10, 38) die Platte durchdringen kann.

15. Analysegerät nach einem der Ansprüche 10 bis 14,
**dadurch gekennzeichnet,**
**dass** das Analysegerät so ausgebildet ist, dass das Beobachtungssystem (13) zusammen mit dem Beleuchtungssystem (10, 38) um eine Geräteachse der Betätigungsvorrichtung (11, 39) bzw. die optische Achse (15, 45) gedreht werden kann.

## Claims

1. An ophthalmological analysis apparatus for measuring an intraocular pressure in an eye, the analysis apparatus comprising an illumination system (10, 38), the analysis apparatus comprising an actuation device (11, 39) with which a puff of air for deforming a cornea (14) can be applied to the eye (22) in a manner aligned with the optical axis (15, 45) of the eye, the illumination system comprising at least one illumination device (23, 40) with which the cornea of the eye can be illuminated by a slit light (25) in such a way that a sectional image (27) can be generated in an illumination plane (26) coinciding with the optical axis,
**characterised in that**
the illumination device is formed and arranged laterally relative to the actuation device in such a way that an illuminating beam path (28, 43) of the illumination device oriented towards the eye is arranged at an angle α relative to the optical axis (15, 45).

2. The analysis apparatus according to claim 1,
**characterised in that**
the illumination system (10, 38) comprises two illumination devices (23, 40) which can illuminate the illumination plane (26).

3. The analysis apparatus according to claim 2,
**characterised in that**
the illumination devices (23, 40) are arranged coaxially to the optical axis (15, 45).

4. The analysis apparatus according to any one of the preceding claims,
**characterised in that**
the illumination device (23, 40) comprises a filter means which can compensate for differences in brightness of a sectional image (27).

5. The analysis apparatus according to claim 4,
**characterised in that**
the filter means forms at least one graduated filter.

6. The analysis apparatus according to claim 5,
**characterised in that**
the graduated filter can shade the illuminating beam path (28, 43) centrally.

7. The analysis apparatus according to either claim 5 or claim 6,
**characterised in that**
the graduated filter can continuously shade the illuminating beam path (28, 43) transverse to the slit light (25).

8. The analysis apparatus according to any one of the preceding claims,
**characterised in that**
a light means of the illumination device (23, 40) is formed of at least one light-emitting diode (30).

9. The analysis apparatus according to any one of the preceding claims,
**characterised in that**
the illumination device (40) comprises a deflection means (42) by means of which the illuminating beam path (43) can be deflected in the illumination device by an angle β.

10. The analysis apparatus according to any one of the preceding claims,
**characterised in that**
the analysis apparatus comprises a monitoring system (13) with which the deformation of the cornea can be monitored and recorded, wherein sectional images (27) of the undeformed and deformed cornea can be recorded using the monitoring system.

11. The analysis apparatus according to claim 10,
**characterised in that**
the illumination system (10, 38) is spatially independent of the actuation device (11, 39).

12. The analysis apparatus according to either claim 10 or claim 11,
**characterised in that**
an illumination device (23, 40) of the illumination system (10, 38) is pivotable by an angle α relative to an apparatus axis of the actuation device (11, 39) or the optical axis (15, 45).

13. The analysis apparatus according to any one of claims 10 to 12,
**characterised in that**
the monitoring system (13) comprises a camera device (32, 33) with which the sectional images (27) can be recorded, and the camera device and the illumination system (10, 38) being arranged in such a way that the camera device and the sectional image are arranged in a Scheimpflug arrangement.

14. The analysis apparatus according to any one of claims 10 to 13,
**characterised in that**
the actuation device (11, 39) comprises a transparent plate (22) in which an opening (21) for outputting the puff of air is formed, an illuminating beam path (28, 43) of the illumination system (10, 38) being able to penetrate through the plate.

15. The analysis apparatus according to any one of claims 10 to 14,
**characterised in that**
the analysis apparatus is formed in such a way that the monitoring system (13), together with the illumination system (10, 38), can be rotated about an apparatus axis of the actuation device (11, 39) or the optical axis (15, 45).

## Revendications

1. Appareil d'analyse ophtalmologique destiné à mesurer une pression intraoculaire dans un oeil, l'appareil d'analyse comprenant un système d'illumination (10, 38), l'appareil d'analyse comprenant un dispositif d'actionnement (11, 39) par lequel un souffle d'air destiné à déformer une cornée (14) peut être appliqué à l'oeil (22) de manière alignée avec l'axe optique (15, 45) de l'oeil, le système d'illumination comprenant au moins un dispositif d'illumination (23, 40) par lequel la cornée de l'oeil peut être illuminée par une fente de lumière (25) d'une telle manière qu'une image en coupe (27) peut être produite dans un plan d'illumination (26) coïncidant avec l'axe optique,
**caractérisé en ce que**
le dispositif d'illumination est formé et disposé latéralement par rapport au dispositif d'actionnement d'une telle manière qu'un chemin optique d'illumination (28, 43) du dispositif d'illumination orienté sur l'oeil est disposé à un angle α par rapport à l'axe optique (15, 45).

2. Appareil d'analyse selon la revendication 1,
**caractérisé en ce que**
le système d'illumination (10, 38) comprend deux dispositifs d'illumination (23, 40) qui peuvent illuminer le plan d'illumination (26).

3. Appareil d'analyse selon la revendication 2,
**caractérisé en ce que**
les dispositifs d'illumination (23, 40) sont disposés de manière coaxiale à l'axe optique (15, 45).

4. Appareil d'analyse selon l'une quelconque des revendications précédentes,
**caractérisé en ce que**
le dispositif d'illumination (23, 40) comprend un moyen de filtre qui peut compenser des différences de luminosité d'une image en coupe (27).

5. Appareil d'analyse selon la revendication 4,
**caractérisé en ce que**
le moyen de filtre forme au moins un filtre dégradé.

6. Appareil d'analyse selon la revendication 5,
**caractérisé en ce que**
le filtre dégradé peut foncer le chemin optique d'illumination (28, 43) au milieu.

7. Appareil d'analyse selon la revendication 5 ou la revendication 6,
**caractérisé en ce que**
le filtre dégradé peut foncer le chemin optique d'illumination (28, 43) de manière continue transversalement à la fente de lumière (25).

8. Appareil d'analyse selon l'une quelconque des revendications précédentes,
**caractérisé en ce qu'**
une lampe du dispositif d'illumination (23, 40) est formé par au moins une diode électroluminescente (30).

9. Appareil d'analyse selon l'une quelconque des revendications précédentes,
**caractérisé en ce que**
le dispositif d'illumination (40) comprend un moyen de déviation (42) par lequel le chemin optique d'illumination (43) peut être dévié par un angle β dans le dispositif d'illumination.

10. Appareil d'analyse selon l'une quelconque des revendications précédentes,
**caractérisé en ce que**
l'appareil d'analyse comprend un system de surveillance (13) par lequel la déformation de la cornée peut être surveillée et enregistrée, des images en coupe (27) de la cornée non déformée et déformée pouvant être enregistrées en utilisant le système de surveillance.

11. Appareil d'analyse selon la revendication 10,
**caractérisé en ce que**
le système d'illumination (10, 38) est spatialement indépendant du dispositif d'actionnement (11, 39).

12. Appareil d'analyse selon la revendication 10 ou la revendication 11,
**caractérisé en ce qu'**
un dispositif d'illumination (23, 40) du système d'illumination (10, 38) est pivotable par un angle α par rapport à un axe d'appareil du dispositif d'actionnement (11, 39) ou par l'axe optique (15, 45).

13. Appareil d'analyse selon l'une quelconque des revendications 10 à 12,
**caractérisé en ce que**
le système de surveillance (13) comprend un dispositif de caméra (32, 33) par lequel les images en coupe (27) peuvent être capturées, le dispositif de caméra et le système d'illumination (10, 38) étant disposés d'une telle manière que le dispositif de caméra et l'image en coupe sont disposés dans une configuration de Scheimpflug.

14. Appareil d'analyse selon l'une quelconque des revendications 10 à 13,
**caractérisé en ce que**
le dispositif d'actionnement (11, 39) comprend une plaque transparente (22) dans laquelle une ouverture (21) pour faire sortir le souffle d'air est formée, un chemin optique d'illumination (28, 43) du système d'illumination (10, 38) pouvant pénétrer la plaque.

15. Appareil d'analyse selon l'une quelconque des revendications 10 à 14,
**caractérisé en ce que**
l'appareil d'analyse est formé d'une telle manière que le système de surveillance (13), conjointement avec le système d'illumination (10, 38), peut être tourné atour d'un axe d'appareil du dispositif d'actionnement (11, 39) ou de l'axe optique (15, 45).
